(19) **Europäisches Patentamt / European Patent Office / Office européen des brevets**

(11) **EP 1 172 334 B1**

(12) **EUROPEAN PATENT SPECIFICATION**

(45) Date of publication and mention
of the grant of the patent:
**21.04.2010 Bulletin 2010/16**

(21) Application number: **00902097.5**

(22) Date of filing: **04.02.2000**

(51) Int Cl.:
***C01G 9/02*** (2006.01)   ***C01G 9/03*** (2006.01)

(86) International application number:
**PCT/JP2000/000621**

(87) International publication number:
**WO 2000/046152 (10.08.2000 Gazette 2000/32)**

(54) **PRODUCTION PROCESS OF AN ULTRAFINE PARTICULATE ZINC OXIDE**

HERSTELLUNGSVERFAHREN VON ULTRAFEINEN ZINKOXIDTEILCHEN

PROCEDE DE PREPARATION DE PARTICULES D'OXYDE DE ZINC

(84) Designated Contracting States:
**AT BE CH CY DE DK ES FI FR GB GR IE IT LI LU MC NL PT SE**

(30) Priority: **05.02.1999 JP 2933999**
**23.02.1999 US 121435 P**

(43) Date of publication of application:
**16.01.2002 Bulletin 2002/03**

(73) Proprietor: **Showa Denko K K**
**Tokyo 105-8518 (JP)**

(72) Inventors:
• **KOGOI, Hisao,**
**Showa Denko K.K.**
**Minato-ku,**
**Tokyo 105-8518 (JP)**
• **TANAKA, Jun,**
**Showa Denko K.K.**
**Minato-ku,**
**Tokyo 105-8518 (JP)**
• **JAMAYA, Hayato,**
**Showa Denko K.K.**
**Minato-ku,**
**Tokyo 105-8518 (JP)**

(74) Representative: **Bradley, Adrian et al**
**Cleveland**
**40-43 Chancery Lane**
**London WC2A 1JQ (GB)**

(56) References cited:
**JP-A- 1 286 919     JP-A- 7 025 614**
**JP-A- 7 118 133     JP-A- 10 167 929**
**US-A- 5 560 871**

• **PATENT ABSTRACTS OF JAPAN vol. 017, no. 285 (C-1066), 2 June 1993 (1993-06-02) & JP 05 017143 A (MITSUI MINING & SMELTING CO LTD), 26 January 1993 (1993-01-26)**
• **PATENT ABSTRACTS OF JAPAN vol. 016, no. 461 (C-0988), 25 September 1992 (1992-09-25) & JP 04 164814 A (MITSUBISHI MATERIALS CORP), 10 June 1992 (1992-06-10)**
• **PATENT ABSTRACTS OF JAPAN vol. 017, no. 079 (C-1027), 17 February 1993 (1993-02-17) & JP 04 280814 A (HAYASHI KINZOKU KOUGIYOUSHIYO:KK), 6 October 1992 (1992-10-06)**

Remarks:
The file contains technical information submitted after the application was filed and not included in this specification

EP 1 172 334 B1

## Description

This application is an application filed under 35 U.S.C. §111(a) claiming the benefit pursuant to 35 U.S.C. §119 (e)(i) of the filing date of Provisional Application 60/121,435 filed February 23, 1999 pursuant to 35 U.S.C. §111(b).

Technical Field:

The present invention relates to a production process of the ultrafine particulate zinc oxide and a cosmetic material using the ultrafine particulate zinc oxide.

The ultrafine particulate zinc oxide that has a low coagulation of primary particles, can be suitably used as cosmetic powder, for example, and is favored with both transparency and ultraviolet-shielding ability. Coagulation can be reduced to such an extent that the particles can be very easily dispersed or suspended in an aqueous solvent even without passing through the process of grinding or after the slight dry grinding.

Background Art:

Many kinds of zinc oxide powders have heretofore been available on the market and applied to an extender blended in cosmetic materials and the like. Known production processes for these zinc oxide powders are roughly classified into a liquid phase process and a gas phase process. In the liquid phase process, zinc oxalate, zinc hydroxide or basic zinc carbonate is synthesized, precipitated, separated by filtration with rinse and then thermally decomposed to obtain zinc oxide. The powder obtained has a specific surface area of 50 $m^2/g$ or more. The liquid phase process is, however, disadvantageous in that the productivity is low because a batch system is fundamentally used and that since the fine particles obtained are in a solid-liquid mixed phase state, they must be subjected to filtration and drying for finishing as a product. Accordingly, the production cost can be hardly reduced. In addition, due to impurities remaining after the synthesis, higher purity cannot be attained. The gas phase process includes a French process of oxidizing zinc vapor and an American process of oxidizing zinc vapor generated at the smelting process of a zinc ore. In this gas phase process, a zinc oxide powder having a specific surface area of 30 $m^2/g$ or more can be obtained. Other than these processes, various proposals for improved processes have been made in recent years with an attempt to further increase the specific surface area and improve the purity.

However, the zinc oxide powder produced by the above-described conventional processes has a problem in that the primary particles are not uniform in shape and size and are readily coagulated to form huge secondary particles. In the case of using such a zinc oxide powder in cosmetic materials or the like, it is necessary to unbind the coagulated particles by cracking or grinding. At this time, mixing of impurities due to abrasion of appliances during the cracking or grinding process inevitably takes place. As a result, not only the purity decreases but also the powder cannot be prevented from lack of uniformity in shape and size of particles. Accordingly, when such a powder is used as a cosmetic material, the cosmetic material suffers from poor touch feeling. Furthermore, due to a large amount of grinding energy required, the cost increases.

An object of the present invention is to provide an ultrafine particulate zinc oxide which has a low coagulation of primary particles and in the case of application to a cosmetic material or the like, can be stably dispersed or suspended as extremely fine particles in an aqueous solvent without passing through any process for unbinding the coagulation, such as cracking or grinding, or after the slight dry grinding and to provide a production process of the ultrafine particulate zinc oxide.

Another object of the present invention is to provide a cosmetic material having both transparency and ultraviolet-shielding ability.

Disclosure of the Invention:

The present invention provides a production process of the ultrafine particulate zinc oxide that comprises, in a reaction of oxidizing zinc vapor in an atmosphere containing oxygen and water vapor, jetting the zinc vapor together with an inert gas serving as a carrier gas into a reactor via a first nozzle and jetting an oxidizing gas containing oxygen and water vapor into the reactor via a second nozzle, thereby inducing a zinc-oxidizing reaction.

Another production process of the ultrafine particulate zinc oxide according to the present invention comprises, in a reaction of oxidizing zinc vapor in an atmosphere containing oxygen and water vapor, jetting the zinc vapor together with an inert gas serving as a carrier gas into a reactor via a first nozzle, jetting an oxidizing gas containing oxygen and water vapor into the reactor via a second nozzle, and jetting into the reactor via a third nozzle an oxidizing gas containing oxygen and water vapor, that is obtained by combustion of a flammable gas, such as propane, hydrogen, etc., with a combustion supporting gas containing an excess amount of oxygen or air, thereby inducing a zinc-oxidizing reaction.

The ultrafine particulate zinc oxide produced by the aforementioned methods has a low coagulation of primary particles and a uniform shape and particle size.

The present invention further provides a cosmetic material **characterized in that** it contains 1 to 40% by weight of the ultrafine particulate zinc oxide and having both transparency and ultraviolet-shielding ability.

The ultrafine particulate zinc oxide may have a specific surface area of from 10 to 200 m$^2$/g that is determined by the single-point BET technique and having a substantially isotropic primary particle shape, wherein the specific volume determined by a tapping machine is from 4 to 40 m$\ell$/g.

The ultrafine particulate zinc oxide exhibits a high ultraviolet-shielding ability owing to its high whiteness and is transparent owing to its high light transmittance in the visible light region. Therefore, it can have extremely good features when used as a cosmetic material.

The ultrafine particulate zinc oxide may have a ratio of 4 or more of the optical density at a wavelength of 370 nm to that at a wavelength of 530 nm, measured after suspending the ultrafine particulate zinc oxide having the specific volume of 4 to 40 m$\ell$/g in polyglyceryl triisostearate.

The ultrafine particulate zinc oxide is excellent particularly in light transmittance in the visible light region.

Brief Description of the Drawings:

Figure 1 is a schematic view showing one example of a reaction apparatus for use in the practice of the production process of the present invention.

Figures 2(a), 2(b) and 2(c) are each a conceptual view showing the reaction apparatus for use in the production process of the present invention.

Figure 3 is a graph showing the values of specific volume/specific surface area in Example Materials of the present invention and Comparative Materials (commercially available products).

Figure 4 is a graph showing the light transmittance in Example Materials of the present invention and Comparative Materials (commercially available products).

Best Mode for Embodying the Invention:

As described above, the conventional zinc oxide powders are readily coagulated to form huge secondary particles. For this reason, it is necessary to unbind the coagulated particles by cracking or grinding. However, this decreases the purity of the powder and cannot prevent the powder from lack of uniformity in shape and size of particles. When using the zinc oxide powder in cosmetic materials or the like, the cosmetic material suffers from poor touch feeling. The inventors have revealed through their detailed studies that the specific volume of the conventional zinc oxide powder is merely as small as 3.5 m$\ell$/g. Upon keen studies on the specific volume of ultrafine particulate zinc oxide obtained without taking a cracking or grinding step that produces powder not uniform in shape and particle size, i.e., so as not to form huge secondary particles, the inventors have found that the ultrafine particulate zinc oxide with a specific volume of 4 to 40 m$\ell$/g obtained by the production process described below has a low coagulation to such an extent that the crushing process is not at all or scarcely necessary and can be instantly used as a cosmetic material. As a result, the present invention has been accomplished.

It has also been found that the ultrafine particulate zinc oxide exhibits a high ultraviolet-shielding ability owing to its high whiteness and is transparent owing to its high light transmittance in the visible light region. Therefore it can have extremely good features when used as a cosmetic material.

The measurement method of the specific volume is described here.

A sample is dried at 105°C to have a constant weight and then passed through a 177 $\mu$m standard sieve. 3.0 g of the powder obtained is precisely weighed and gently placed in a 20 m$\ell$ test tube with graduations. This test tube is mounted in a metal tube. The metal tube is covered and then dropped 400 times from the height of 45 mm at a rate of once per 2 seconds. Thereafter, the volume (m$\ell$) is read and V obtained by the expression: V = volume (m$\ell$)/3 (g) is defined as the specific volume of the sample.

The ultraviolet-shielding ability is described here.

20 g of polyglyceryl triisostearate, 200 mg of a powder (sample) dried at 105°C to have a constant weight, 200 g of 1 mm$\phi$ ZrO$_2$ beads and 10 pieces of 10 mm$\phi$ ZrO$_2$ beads as mixing media are charged into a vessel and mixed in a bench ball mill at 100 rpm for 30 minutes. The optical density (corresponding to the reciprocal of the light transmittance) of the mixture obtained is measured at respective wavelengths with a spectrophotometer for ultraviolet and visible region. The cell used is a 0.1 mm quartz cell and the wavelength of measurement is from 200 to 800 nm. Of course, blank correction of polyglyceryl triisostearate is performed. Among the values determined, the ratio of the optical density at a wavelength of 370 nm to the optical density at 530 nm is used as an index of the ultraviolet-shielding ability and the transparency (light transmittance in the visible light region).

In the case of conventional zinc oxide powders, the ratio of the optical density at a wavelength of 370 nm to the optical density at 530 nm is only on the order of from 1 to 3. On the other hand, in the ultrafine particulate zinc oxide of the present invention, the ratio of the optical density at a wavelength of 370 nm to the optical density at 530 nm is 4 or more. Thus, it can be seen that the light transmittance in the visible light region is particularly excellent as compared with that of the conventional powders.

The present invention provides a process for producing an ultrafine particulate zinc oxide having the above-described properties which conventional powders cannot possess, the process comprising oxidizing a zinc vapor in an atmosphere containing oxygen and water vapor, wherein the zinc vapor is jetted out from a first nozzle into a reactor together with an inert gas serving as a carrier gas, and an oxidizing gas containing oxygen and water vapor is jetted out from a second nozzle into the reactor, to cause oxidation reaction of zinc.

The present invention also provides a process for producing an ultrafine particulate zinc oxide having the above-described properties which conventional powders cannot possess, the process comprising oxidizing zinc vapor in an atmosphere containing oxygen and water vapor, wherein the zinc vapor is jetted out from a first nozzle into a reactor together with an inert gas serving as a carrier gas, an oxidizing gas containing oxygen and water vapor is jetted out from a second nozzle into the reactor, and an oxidizing gas obtained by the combustion of a flammable gas such as propane or hydrogen with an excess combustion supporting gas such as oxygen or air is jetted out from a third nozzle into the reactor, to cause oxidation reaction of zinc.

As a result of extensive investigations on the production of ultrafine particulate zinc oxide according to the gas phase process, it has been found that when zinc is oxidized by jetting out a raw material gas comprising zinc vapor beforehand vaporized and an inert gas serving as the carrier gas from a first nozzle into a reactor, jetting out an oxidizing gas containing oxygen and water vapor from a second nozzle into the reactor, and/or jetting out an oxidizing gas obtained by the combustion of a flammable gas such as propane or hydrogen with an excess combustion supporting gas such as oxygen or air from the second nozzle or a third nozzle, an ultrafine particulate zinc oxide having a low coagulation of primary particles and with a uniform shape and particle size can be produced.

The oxidizing gas containing oxygen and water vapor may be an oxidizing gas obtained by the combustion of a flammable gas such as propane or hydrogen with an excess combustion supporting gas such as oxygen or air. Furthermore, a plurality of nozzles may be used for each jetting of the oxidizing gas and the raw material gas.

The temperature used when jetting out the zinc vapor from the first nozzle together with an inert gas serving as the carrier gas is from 900 to 1,800°C, preferably from 1,000 to 1,500°C.

The temperature used when jetting out the oxidizing gas containing oxygen and water vapor from the second or third nozzle is from 900 to 1,200°C, preferably from 1,000 to 1,200°C.

In the stream conveying the oxidizing gas containing oxygen and water vapor jetted out from the second or third nozzle, the oxygen concentration is from 5 to 100 vol%, preferably from 50 to 100 vol%. The total of the oxygen concentration and the water vapor concentration in this stream is from 5 to 100 vol%.

At the time of jetting out the zinc vapor from the first nozzle together with an inert gas serving as the carrier gas, the jet velocity is from 10 to 200 m/sec, preferably from 15 to 100 m/sec.

In jetting out the oxidizing gas containing oxygen and water vapor from the second or third nozzle, the jet velocity is preferably from 2 to 250 m/sec.

After the oxidation reaction of zinc, the temperature is preferably controlled to a range where condensation of water does not occur. The temperature where condensation of water does not occur is 100°C or more, preferably from 100 to 150°C.

On taking notice of the V/A value obtained from a specific volume V and a specific surface area A of the ultrafine particulate zinc oxide produced by the above-described production process, it has been found that in conventional zinc oxide powder, this value is small and is less than 0.15, but in the present invention, the V/A value is very large and is 0.18 or more.

Furthermore, the ultrafine particulate zinc oxide produced by the above-described production process has a tendency to exhibit a unique property such that the V/A value lies in the range of from (Y value + 200%) to (Y value - 30%), the Y value being obtained by the following equation (1):

$$Y = V/A = -0.152 Ln(A) + 0.745 \tag{1}$$

(wherein Ln(A) represents a natural logarithm of the specific surface area (unit: $m^2/g$)).

The production process of the present invention is described below by referring to the drawings attached hereto.

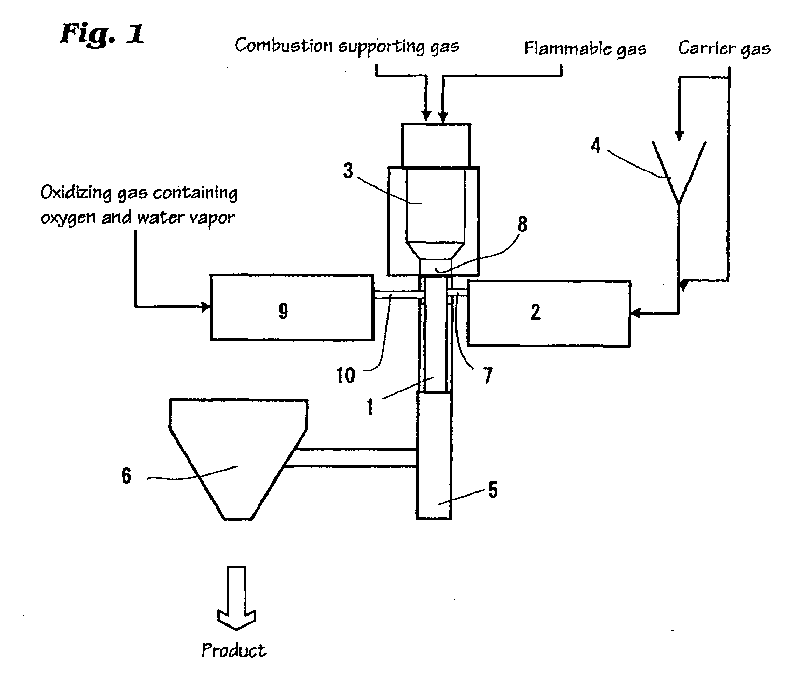

Figure 1 is a schematic view showing one example of an apparatus suitably used for the production of ultrafine particulate zinc oxide of the present invention. In Figure 1, the raw material zinc fed into a hopper 4 is transferred to a zinc vaporizer 2 by a carrier gas, and the zinc vapor generated in the zinc vaporizer 2 is introduced by an inert gas into a reactor 1 through a first nozzle 7.

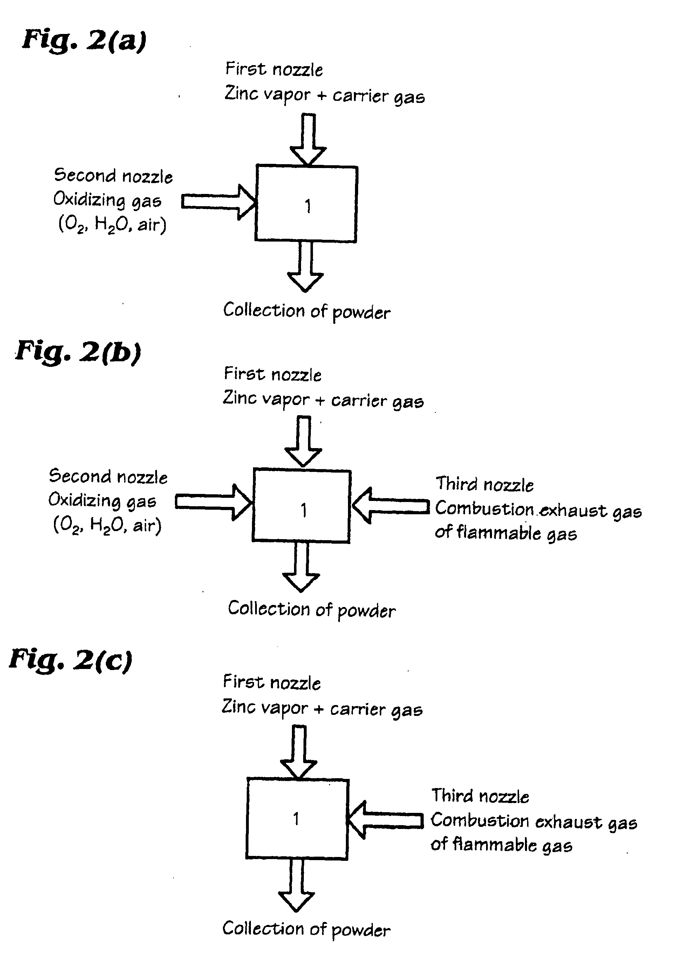

On the other hand, an oxidizing gas containing oxygen and water vapor is introduced into the gas heater 9, and the heated gas is introduced into the reactor 1 through a second nozzle 10 and/or an oxidizing gas obtained by the combustion of a flammable gas such as propane or hydrogen with an excess combustion supporting gas such as oxygen or air occurring in a combustion chamber 3 is introduced into the reactor 1 through a third nozzle 8, whereby zinc is oxidized (see, for example, Figures 2(a) to 2(c)).

This reaction stops by a cooling process where cooling air is blown onto the zinc oxide transferred to a cooler 5.

The shape and size of the zinc oxide particles obtained can be controlled by varying the length of the reaction field, the residence time or the like. Thereafter, zinc oxide is collected as a product by a collecting means (unit) 6 such as a bag filter or cyclone.

In the present invention, the ultrafine particulate zinc oxide powder is coated with silica when necessary. The method for forming a silica coating is not particularly limited and the method described in WO98/47476, for example, may be used.

However, the silica coating can be fundamentally formed by dipping the ultrafine particulate zinc oxide powder in a composition for forming a silica coating and keeping it at a predetermined temperature to selectively deposit silica on the surface of the zinc oxide powder. A method of charging the zinc oxide powder into a composition for forming a coating prepared in advance to form a silica coating, or a method of suspending the zinc oxide powder beforehand in a solvent and adding thereto other raw material components to make a composition for forming a coating and thereby form a silica coating, may also be used. In other words, the order in charging the raw materials of the composition for forming a coating and the ultrafine particulate zinc oxide powder is not particularly limited and the coating can be formed whichever is charged earlier.

The ultrafine particulate zinc oxide powder and the silica-coated ultrafine particulate zinc oxide powder of the present invention may be employed over a wide range of products, other than the cosmetic materials, that include pigments, ultraviolet-shielding materials and photo-catalysts with controlled activity, for example.

The cosmetic material of the production process of the present invention contains the ultrafine particulate zinc oxide powder and/or silica-coated ultrafine particulate zinc oxide powder and may be produced by a conventional production process additionally using commonly used raw materials which can be blended in cosmetic materials. The cosmetic material of the present invention contains 1 to 40 mass% of ultrafine particulate zinc oxide powder or 1 to 40 mass% of silica-coated ultrafine particulate zinc oxide powder. On considering the ultraviolet-shielding effect, the content of the ultrafine particulate zinc oxide powder and/or silica-coated ultrafine particulate zinc oxide powder is preferably from 3 to 25 mass%, more preferably from 5 to 20 mass%.

If the content of the ultrafine particulate zinc oxide powder and/or silica-coated ultrafine particulate zinc oxide powder is less than 1 mass%, a sufficiently high ultraviolet-shielding effect may not be obtained, whereas if it exceeds 40 mass%, formulation may not be formed.

The cosmetic material of the production process of the present invention is not particularly limited so long as it contains the powder, but includes those obtained by dispersing the powder in a solvent or a solution. Examples of cosmetic material containing the powder include cosmetic materials in the form of powder, press, stick or liquid. Specific examples thereof include face powder, foundation, cosmetic powder, cheek color products, eye shadow, lipstick, eyeliner, mascara and eyebrow products. Specific examples of cosmetic material obtained by dispersing the powder in a solvent or a solution include cream, essence, lotion, skin lotion, milky lotion and mousse. In particular, the ultrafine particulate zinc oxide powder and/or silica-coated ultrafine particulate zinc oxide powder of the present invention is preferably used for solid powder cosmetics.

The present invention is described in greater detail below by referring to the Examples and Comparative Examples.

(Evaluation Method of Physical Properties)

The physical properties were measured and evaluated using the following methods.

For the X-ray diffraction, an apparatus, Model 2000/PC, manufactured by Rigaku KK was used.

The specific surface area was measured in the conditions for the single-point BET technique using a Monosorb-type apparatus manufactured by Quantachrome Corporation.

The specific volume was measured by the method according to the Standards for Cosmetic Ingredients using a tapping machine manufactured by Kuramochi Kagaku Kiki Seisakusho.

For the initial evaluation of transparency of the zinc oxide (sample), 200 mg of the sample was suspended in 20 g of polyglyceryl triisostearate (COSMOL-43, produced by The Nisshin Oil Mills Ltd.) using a bench ball mill V-1M manufactured by Irie Seisakusho Ltd., and light transmittance at a wavelength of from 280 to 700 nm was measured by a predetermined method using a spectrophotometer for the ultraviolet and visible region, UV-160 manufactured by Shimadzu Corporation.

(Example 1)

An ultrafine particulate zinc oxide was produced using the reaction apparatus shown in Figure 1 (this applies to following Examples 2 to 4).

A container having a large heat transfer surface area such that zinc vaporizes in an amount of 9 kg/hr was heated up to 1,150°C, then nitrogen serving as the carrier gas was blown into the container at 4 Nm$^3$/hr, and the vapor generated was introduced into a reaction tube from a nozzle 1 under thermal insulation. On the other hand, air and water at flow rates of 20 Nm$^3$/hr and 700 m$\ell$/hr, respectively, were heated to 1,100°C, introduced into the reaction tube from a nozzle 2 and reacted with the above-described raw material gas. The white powder obtained was examined by X-ray diffraction and found to be Zincite. Thus, the powder was identified as zinc oxide. The more particular production conditions are shown in Table 1.

The physical properties of the thus-obtained ultrafine particulate zinc oxide (Example Material 1) are shown in Table 2.

(Example 2)

A container having a large heat transfer surface area such that zinc vaporizes in an amount of 4 kg/hr was heated up to 1,100°C, then nitrogen serving as the carrier gas was blown into the container at 1 Nm$^3$/hr, and the vapor generated was introduced into a reaction tube from a nozzle 1 under thermal insulation. On the other hand, oxygen and water at flow rates of 40 Nm$^3$/hr and 6 $\ell$/hr, respectively, were heated to 1,100°C, introduced into the reaction tube from a nozzle 2 and reacted with the above-described raw material gas. The white powder obtained was examined by X-ray diffraction and was found to be Zincite. Thus, the powder was identified as zinc oxide. The more particular production conditions are shown in Table 1.

The physical properties of the thus-obtained ultrafine particulate zinc oxide (Example Material 2) are shown in Table 2.

(Example 3)

A container having a large heat transfer surface area such that zinc vaporizes in an amount of 10 kg/hr was heated up to 1,100°C, then nitrogen serving as the carrier gas was blown into the container at 2 Nm$^3$/hr, and the vapor generated was introduced into a reaction tube from a nozzle 1 under thermal insulation. On the other hand, oxygen and water at flow rates of 120 Nm$^3$/hr and 15 $\ell$/hr, respectively, were heated to 1,150°C, introduced into the reaction tube from a nozzle 2 and reacted with the above-described raw material gas. The white powder obtained was examined by X-ray diffraction and found to be Zincite. Thus, the powder was identified as zinc oxide. The more particular production conditions are shown in Table 1.

The physical properties of the thus-obtained ultrafine particulate zinc oxide (Example Material 3) are shown in Table 2.

(Example 4)

A container having a large heat transfer surface area such that zinc vaporizes in an amount of 6 kg/hr was heated up to 1,150°C, then nitrogen serving as the carrier gas was blown into the container at 2 Nm$^3$/hr, and the vapor generated was introduced into a reaction tube from a nozzle 1 under thermal insulation. On the other hand, oxygen and water at flow rates of 20 Nm$^3$/hr and 400 m$\ell$/hr, respectively, were heated to 1,150°C, introduced into the reaction tube from a nozzle 2 and reacted with the above-described raw material gas. The white powder obtained was examined by X-ray diffraction and found to be Zincite. Thus, the powder was identified as zinc oxide. The more particular production conditions are shown in Table 1.

The physical properties of the thus-obtained ultrafine particulate zinc oxide (Example Material 4) are shown in Table 2.

(Table 1)

| Production Condition | Example 1 | Example 2 | Example 3 | Example 4 |
|---|---|---|---|---|
| Temperature of raw material gas (°C) | 1,150 | 1,100 | 1,150 | 1,150 |
| Amount of Zn vaporized (kg/hr) | 9 | 4 | 10 | 6 |
| Amount of carrier gas fed (Nm$^3$/hr) | | 4 1 | | 2 |
| Zn concentration in raw material gas (vol%) | 44 | 58 | 63 | 51 |

(continued)

| Production Condition | Example 1 | Example 2 | Example 3 | Example 4 |
|---|---|---|---|---|
| Flow rate of oxidizing gas, Air (Nm$^3$/hr) | 20 | 0 | | 0 |
| Flow rate of oxidizing gas, $O_2$ (Nm$^3$/hr) | 0 | 40 | 120 | 20 |
| Amount of water vapor, $H_2O$ (g/hr) | 700 | 6,000 | 15,000 | 400 |
| Total flow rate of oxidizing gas (Nm$^3$/hr) | 21 | 47 | 139 | 20 |
| Temperature of oxidizing gas (°C) | 1,100 | 1,100 | 1,150 | 1,150 |
| Total of $O_2$ concentration and $H_2O$ concentration in oxidizing gas (%) | 24 | 100 | 100 | 100 |
| Flow rate of raw material gas jetted out from nozzle (m/s) | 33 | 11 | 100 | 19 |
| Flow rate of oxidizing gas jetted out from nozzle (m/s) | 23 | 53 | 160 | 24 |
| Oxygen excess (%) | 273 | 5,839 | 7,007 | 1,946 |
| Collection temperature (°C) | 120 | 130 | 130 | 130 |

Definition of Oxygen Excess in the Table:

$$\frac{\text{Amount of Oxygen Gas in Oxidizing Gas}}{\text{Theoretical Amount of Oxygen Gas Necessary for Reaction}} \times 100$$

(Comparative Materials A to D)

Four kinds of commercially available zinc oxide powders, namely, ZnO-350 produced by Sumitomo Osaka Cement Co., Ltd. (Comparative Material A), FINEX-50 produced by Sakai Chemical Industry Co., Ltd. (Comparative Material B), Z-COTE produced by sunSmart Inc. (Comparative Material C) and USP-1 produced by Zinc Corporation of America (Comparative Material D), were selected and the physical properties thereof are shown in Table 2.

(Table 2)

| | Example Material 1 | Example Material 2 | Example Material 3 | Example Material 4 | Comparative Material A | Comparative Material B | Comparative Material C | Comparative Material D |
|---|---|---|---|---|---|---|---|---|
| Light transmittance | See Figure 3 | See Figure 3 | See Figure 3 | See Figure 3 | See Figure 3 | See Figure 3 | See Figure 3 | See Figure 3 |
| OD370 | 1.064 | 1.002 | 1.193 | 1.119 | 1.01 | 0.895 | 0.754 | 0.693 |
| OD530 | 0.263 | 0.239 | 0.246 | 0.338 | 0.397 | 0.24 | 0.416 | 0.402 |
| OD370/OD530 | 4.05 | 4.19 | 4.85 | 3.31 | 2.54 | 3.73 | 1.81 | 1.72 |
| Primary particle size (nm) | 48 | 44 | 31 | 76 | 35 | 30 | 57 | 130 |
| Mean particle size ($\mu$m) | 0.48 | 0.52 | 0.47 | 0.55 | 1.7 | 2.57 | 1.08 | 0.47 |
| Specific volume V (ml/g) | 6 | 7.1 | 6.1 | 4.65 | 2.35 | 2.5 | 1.55 | 1.23 |
| Specific surface area A ($m^2$/g) | 22 | 24 | 34 | 14 | 30 | 34.8 | 18.6 | 8.1 |
| V/A ($m\ell/m^2$) | 0.27 | 0.30 | 0.18 | 0.33 | 0.08 | 0.07 | 0.08 | 0.15 |
| L value by Hunter color tester | 94.2 | 93.8 | 93.5 | 94.6 | | | | |

(Evaluation of Physical Properties)

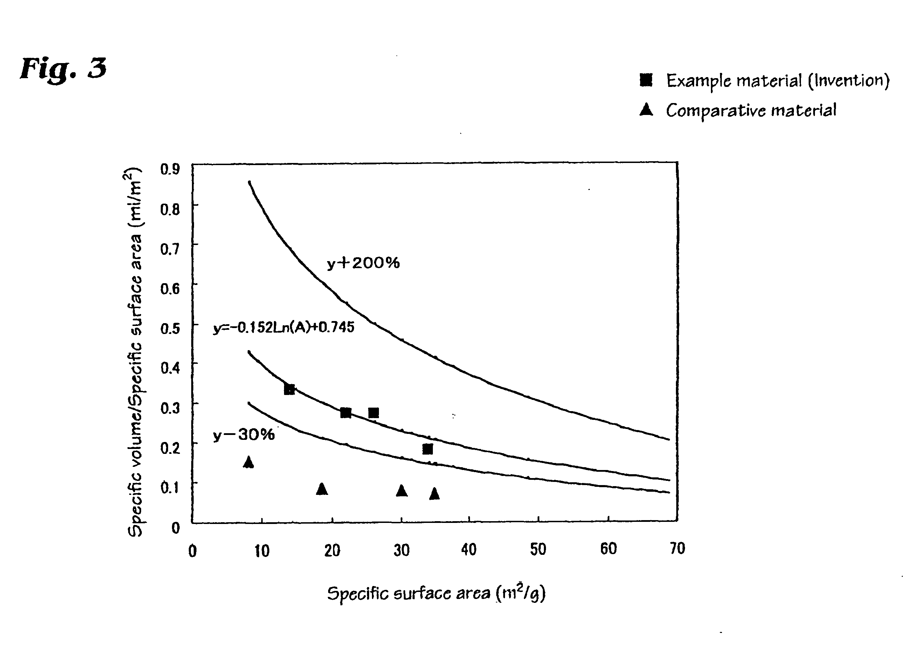

As is apparent from Table 2, each of Example Materials 1 to 4 of the present invention was an ultrafine particulate zinc oxide powder having a large specific volume and a small bulk specific gravity. Furthermore, all the ultrafine particulate zinc oxides, i.e. Example Materials 1 to 4 of the present invention, had a value of specific volume V/specific surface area A of 0.18 or more, and the values were very large as compared with Comparative Materials A to D which were commercially available zinc oxide powders (see Figure 3). The tendency in the physical properties was analyzed by plotting the V/A values with respect to the specific surface area. As a result, it was surprisingly found that the group of Example Materials 1 to 4 of the present invention and the group of Comparative Materials A to D were present in the vicinity of respective peculiar asymptotic curves. Example Materials 1 to 4 of the present invention were present in the range of from +200% to -30% of the curve value (V/A) obtained according to the formula V/A = -0.152Ln(A) + 0.745 (wherein Ln(A) represents a natural logarithm of a specific surface area). This tendency is unique as compared with the characteristic behaviors (curves) of Comparative Materials.

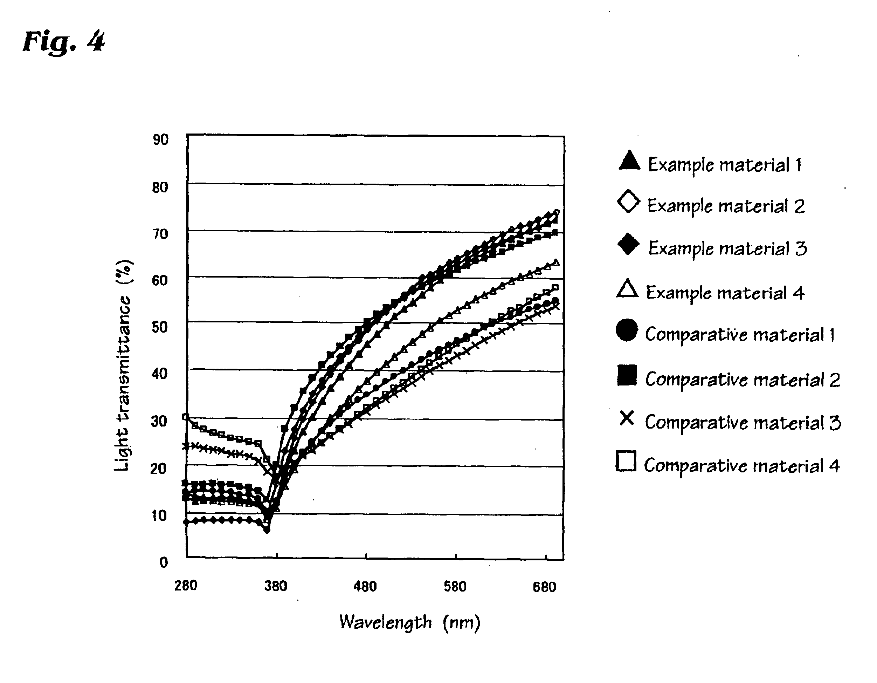

Furthermore, when an ultraviolet and visible light absorption spectrum of the dispersion system was measured, Example Materials 1 to 4 showed unique transparency as compared with Comparative Materials A to D (see Figure 4). In particular, Example Materials 1 to 4 were unique in that the ratio of the optical density at the wavelength of 370 nm (OD370) to the optical density at the wavelength of 530 nm (OD530) was 4 or more. Example Materials 1 to 4 were also unique with respect to the L value determined by a Hunter color tester.

The coating of silica on the ultrafine particulate zinc oxide powder and the formulation of a foundation using this silica-coated zinc oxide powder are described in the following Examples.

(Example 5)

In a 5 ℓ-volume reactor, 991 mℓ of deionized water, 1,083 mℓ of ethanol (produced by Junsei Chemical Co., Ltd.) and 6.7 mℓ of a 25 weight% aqueous ammonia (produced by Taisei Kako Co., Ltd.) were mixed, and therein 67 g of zinc oxide powder (MZ0350 produced by Sumitomo Osaka Cement; primary particle size: 37 nm) was dispersed to prepare a suspension. Separately, 135 mℓ of tetraethoxysilane (produced by Nakarai Tesque, Inc. Laboratory) and 60 mℓ of ethanol were mixed to prepare a solution.

To the suspension under stirring with a stirrer, the solution was added at a constant rate for 8.5 hours. The resulting mixed solution was ripened for 12 hours. The formation and ripening of the silica coating were performed at a pH of 10.5 and a temperature of 35°C. Thereafter, the solid contents were separated by centrifugal filtration and vacuum-dried at 50°C for 12 hours to obtain silica-coated ultrafine particulate zinc oxide powder.

(Example 6)

A foundation having the following formulation was produced by a conventional method. As the silica-coated ultrafine particulate metal oxide powder, the silica-coated ultrafine particulate metal oxide powder obtained in Example 5 was used.

Formulation of Foundation:

| | |
|---|---|
| Fine particulate titanium oxide | 10.0 mass% |
| Silica-coated ultrafine particulate zinc oxide powder | 15.0 mass% |
| Mica | 20.0 mass% |
| Talc | 10.0 mass% |
| Zinc white | 5.0 mass% |
| Iron oxide (red) | optimum |
| Iron oxide (yellow) | optimum |
| Glycerin | 10.0 mass% |
| Purified water | 30.0 mass% |
| Perfume | optimum |

Industrial Applicability:

As described in the foregoing, the ultrafine particulate zinc oxide of the production process of the present invention has a low coagulation of primary particles and can be very easily dispersed or suspended in an aqueous solvent even without passing through the process of grinding or after the slight dry grinding. Therefore, this ultrafine particulate zinc oxide can be suitably used, for example, for cosmetic materials and in this case, can impart transparency and ultraviolet-shielding ability to the cosmetic material.

The production process of ultrafine particulate zinc oxide of the present invention has a very high practical value because ultrafine particulate zinc oxide having the above-described functions and effects can be continuously produced without using specific facilities or chemicals.

Furthermore, the cosmetic material of the production process of the present invention has a very high practical value because it has high transparency and excellent in ultraviolet-shielding ability.

**Claims**

1.    A process for producing an ultrafine particulate zinc oxide, comprising the steps of oxidizing zinc vapor in an atmosphere containing oxygen and water vapor by jetting the zinc vapor from a first nozzle into a reactor together with an inert gas serving as a carrier gas, and jetting an oxidizing gas containing oxygen and water vapor from a second nozzle into the reactor, to cause an oxidation reaction of zinc.

2.    A process for producing an ultrafine particulate zinc oxide, comprising the steps of oxidizing zinc vapor in an atmosphere containing oxygen and water vapor by jetting zinc vapor from a first nozzle into a reactor together with an inert gas serving as a carrier gas, jetting an oxidizing gas containing oxygen and water vapor from a second nozzle into the reactor, and jetting an oxidizing gas containing oxygen and water vapor, obtained by combustion of a flammable gas with an excess combustion supporting gas, from a third nozzle into the reactor, to cause an oxidation reaction of zinc.

3.    The process for producing an ultrafine particulate zinc oxide as claimed in claim 1 or 2, wherein the step of jetting the zinc vapor from the first nozzle together with the inert gas serving as the carrier gas is performed at a temperature of 900 to 1,200 °C.

4.    The process for producing an ultrafine particulate zinc oxide as claimed in any one of claims 1 to 3, wherein the step of jetting the zinc vapor from the first nozzle together with the inert gas serving as the carrier gas is performed at a jet velocity of 10 to 200 m/sec.

5.    The process for producing an ultrafine particulate zinc oxide as claimed in any one of claims 1 to 4, wherein the oxidizing gas containing oxygen and water vapor is obtained by combustion of a flammable gas with an excess combustion supporting gas.

6.    The process for producing an ultrafine particulate zinc oxide as claimed in any one claims 1 to 5, wherein the step of jetting the oxidizing gas containing oxygen and water vapor from the second or third nozzle is performed at a temperature of 900 to 1,800 °C.

7.    The process for producing an ultrafine particulate zinc oxide as claimed in any one of claims 1 to 6, wherein the step of jetting the oxidizing gas containing oxygen and water vapor from the second or third nozzle is performed at a jet velocity of 2 to 250 m/sec.

8.    The process for producing an ultrafine particulate zinc oxide as claimed in any one of claims 1 to 7, wherein an oxygen concentration in the oxidizing gas containing oxygen and water vapor jetted from the second or third nozzle is 5 to 100 vol% and the total of the oxygen concentration and a water vapor concentration is not less than 5 vol% and less than 100 vol%.

9.    The process for producing an ultrafine particulate zinc oxide as claimed in any one of claims 1 to 8, wherein after the oxidation reaction of zinc, the process further comprises the step of controlling a temperature to a range free of condensation of water.

10.    The process for producing an ultrafine particulate zinc oxide as claimed in claim 9, wherein the temperature free of condensation of water is 100 °C or more.

11. The process for producing an ultrafine particulate zinc oxide as claimed in any one of claims 1 to 10, wherein the nozzle for jetting the oxidizing gas comprises a plurality of nozzles.

12. The process for producing an ultrafine particulate zinc oxide as claimed in any one of claims 1 to 11, comprising the further step of coating silica onto the surface of the ultrafine particulate zinc oxide.

13. The process for producing an ultrafine particulate zinc oxide as claimed in any one of claims 1 to 11 comprising the further step of formulating the ultrafine particulate zinc oxide as a cosmetic material, wherein the cosmetic material comprises ultrafine particulate zinc oxide in an amount of 1 to 40 %.

**Patentansprüche**

1. Ein Verfahren zum Herstellen eines ultrafeinen partikelförmigen Zinkoxids, das die Schritte umfasst: Oxidieren von Zinkdampf in einer Atmosphäre, die Sauerstoff und Wasserdampf enthält, mittels Ausstoßen des Zinkdampfs aus einer ersten Düse in einen Reaktor zusammen mit einem Inertgas, das als Trägergas dient, und Ausstoßen eines Oxidationsgases aus einer zweiten Düse in den Reaktor, welches Sauerstoff und Wasserdampf enthält, um eine Oxidationsreaktion des Zinks zu bewirken.

2. Ein Verfahren zum Herstellen eines ultrafeinen partikelförmigen Zinkoxids, das die Schritte aufweist: Oxidieren von Zinkdampf in einer Atmosphäre, die Sauerstoff und Wasserdampf enthält, mittels Ausstoßen von Zinkdampf aus einer ersten Düse in einen Reaktor zusammen mit einem Inertgas, das als ein Trägergas dient, Ausstoßen eines Oxidationsgases aus einer zweiten Düse in den Reaktor, welches Sauerstoff und Wasserdampf enthält, und Ausstoßen eines Oxidationsgases aus einer dritten Düse in den Reaktor, welches Sauerstoff und Wasserdampf enthält und welches mittels Verbrennung eines brennbaren Gases mit einem Überschussverbrennungsunterstützungsgas erhalten wurde, um eine Oxidationsreaktion des Zinks zu bewirken.

3. Das Verfahren zum Herstellen eines ultrafeinen partikelförmigen Zinkoxids gemäß Anspruch 1 oder 2, während der Schritt des Ausstoßens von Zinkdampf aus der ersten Düse zusammen mit dem Inertgas, das als ein Trägergas dient, bei einer Temperatur von 900 bis 1200 °C durchgeführt wird.

4. Das Verfahren zum Herstellen eines ultrafeinen partikelförmigen Zinkoxids gemäß einem der Ansprüche 1 bis 3, während der Schritt des Ausstoßens von Zinkdampf aus der ersten Düse zusammen mit dem Inertgas, das als das Trägergas dient, bei einer Ausstoßgeschwindigkeit von 10 bis 200 m/s durchgeführt wird.

5. Das Verfahren zum Herstellen eines ultrafeinen partikelförmigen Zinkoxids gemäß einem der Ansprüche 1 bis 4, während das Oxidationsgas, das Sauerstoff und Wasserdampf enthält, mittels Verbrennung eines brennbaren Gases mit einem Überschussverbrennungsunterstützungsgas erhalten wird.

6. Das Verfahren zum Herstellen eines ultrafeinen partikelförmigen Zinkoxids gemäß einem der Ansprüche 1 bis 5, in dem der Schritt des Ausstoßens des Oxidationsgases, das Sauerstoff und Wasserdampf enthält, aus der zweiten oder dritten Düse bei einer Temperatur von 900 bis 1800 °C durchgeführt wird.

7. Das Verfahren zum Herstellen eines ultrafeinen partikelförmigen Zinkoxids gemäß einem der Ansprüche 1 bis 6, während der Schritt des Ausstoßens des Oxidationsgases, das Sauerstoff und Wasserdampf enthält, aus der zweiten oder dritten Düse bei einer Ausstoßgeschwindigkeit von 2 bis 250 m/s durchgeführt wird.

8. Das Verfahren zum Herstellen eines ultrafeinen partikelförmigen Zinkoxids gemäß einem der Ansprüche 1 bis 7, während eine Sauerstoffkonzentration in dem Oxidationsgas, das Sauerstoff und Wasserdampf enthält und das von der zweiten oder dritten Düse ausgestoßen wird, 5 bis 100 Vol.-% beträgt und der Gesamtbetrag der Sauerstoffkonzentration und Wasserdampfkonzentration ist nicht geringer als 5 Vol.-% und niedriger als 100 Vol.-%.

9. Das Verfahren zum Herstellen eines ultrafeinen partikelförmigen Zinkoxids gemäß einem der Ansprüche 1 bis 8, während nach der Oxidationsreaktion des Zinks das Verfahren weiterhin den Schritt aufweist: Steuern einer Temperatur in einen Bereich, der frei von Wasserkondensation ist.

**10.** Das Verfahren zum Herstellen eines ultrafeinen partikelförmigen Zinkoxids gemäß Anspruch 9, in dem die Temperatur, die frei von Wasserkondensation ist, 100 °C oder mehr beträgt.

**11.** Das Verfahren zum Herstellen eines ultrafeinen partikelförmigen Zinkoxids gemäß einem der Ansprüche 1 bis 10, während die Düse zum Ausstoßen des Oxidationsgases eine Mehrzahl an Düsen umfasst.

**12.** Das Verfahren zum Herstellen eines ultrafeinen partikelförmigen Zinkoxids gemäß einem der Ansprüche 1 bis 11, das den weiteren Schritt aufweist: Beschichten der Oberfläche des ultrafeinen partikelförmigen Zinkoxids mit Silica.

**13.** Das Verfahren zum Herstellen eines ultrafeinen partikelförmigen Zinkoxids gemäß einem der Ansprüche 1 bis 11, das weiterhin den Schritt umfasst: Formulieren des ultrafeinen partikelförmigen Zinkoxids als ein kosmetisches Material, während das kosmetische Material ultrafeines partikelförmiges Zinkoxid in einer Menge von 1 bis 40 % aufweist.

**Revendications**

**1.** Procédé pour produire un oxyde de zinc particulaire ultrafin, comprenant les étapes consistant à oxyder de la vapeur de zinc dans une atmosphère contenant de l'oxygène et de la vapeur d'eau par éjection d'un jet de vapeur de zinc depuis une première buse dans un réacteur conjointement avec un gaz inerte servant de gaz vecteur, et éjecter un jet de gaz oxydant contenant de l'oxygène et de la vapeur d'eau depuis une deuxième buse dans le réacteur, pour causer une réaction d'oxydation de zinc.

**2.** Procédé pour produire un oxyde de zinc particulaire ultrafin, comprenant les étapes consistant à oxyder de la vapeur de zinc dans une atmosphère contenant de l'oxygène et de la vapeur d'eau par éjection d'un jet de vapeur de zinc depuis une première buse dans un réacteur conjointement avec un gaz inerte servant de gaz vecteur, éjecter un jet de gaz oxydant contenant de l'oxygène et de la vapeur d'eau depuis une deuxième buse dans le réacteur, et éjecter un jet d'un gaz oxydant contenant de l'oxygène et de la vapeur d'eau, obtenu par combustion d'un gaz inflammable avec un excès de gaz soutenant la combustion, depuis une troisième buse dans le réacteur, pour causer une réaction d'oxydation de zinc.

**3.** Procédé pour produire un oxyde de zinc particulaire ultrafin selon la revendication 1 ou 2, **caractérisé en ce que** l'étape d'éjection d'un jet de vapeur de zinc depuis la première buse conjointement avec le gaz inerte servant de gaz vecteur est effectuée à une température de 900 à 1 200 °C.

**4.** Procédé pour produire un oxyde de zinc particulaire ultrafin selon l'une quelconque des revendications 1 à 3, **caractérisé en ce que** l'étape d'éjection d'un jet de vapeur de zinc depuis la première buse conjointement avec le gaz inerte servant de gaz vecteur est effectuée à une vitesse de jet de 10 à 200 m/s.

**5.** Procédé pour produire un oxyde de zinc particulaire ultrafin selon l'une quelconque des revendications 1 à 4, **caractérisé en ce que** le gaz oxydant contenant de l'oxygène et de la vapeur d'eau est obtenu par combustion d'un gaz inflammable avec un excès de gaz soutenant la combustion.

**6.** Procédé pour produire un oxyde de zinc particulaire ultrafin selon l'une quelconque des revendications 1 à 5, **caractérisé en ce que** l'étape consistant à éjecter un jet du gaz oxydant contenant de l'oxygène et de la vapeur d'eau depuis la deuxième ou troisième buse est effectuée à une température de 900 à 1 800 °C.

**7.** Procédé pour produire un oxyde de zinc particulaire ultrafin selon l'une quelconque des revendications 1 à 6, **caractérisé en ce que** l'étape consistant à éjecter un jet du gaz oxydant contenant de l'oxygène et de la vapeur d'eau depuis la deuxième ou troisième buse est effectuée à une vitesse de jet de 2 à 250 m/s.

**8.** Procédé pour produire un oxyde de zinc particulaire ultrafin selon l'une quelconque des revendications 1 à 7, **caractérisé en ce que** la concentration d'oxygène dans le gaz oxydant contenant de l'oxygène et de la vapeur d'eau éjectée depuis la deuxième ou troisième buse est de 5 à 100 % en volume et le total de la concentration d'oxygène et la concentration de vapeur d'eau n'est pas inférieur à 5 % en volume et inférieur à 100 % en volume.

**9.** Procédé pour produire un oxyde de zinc particulaire ultrafin selon l'une quelconque des revendications 1 à 8, **caractérisé en ce qu'**après la réaction d'oxydation de zinc, le procédé comprend en outre l'étape consistant à contrôler la température dans une plage sans condensation d'eau.

10. Procédé pour produire un oxyde de zinc particulaire ultrafin selon la revendication 9, **caractérisé en ce que** la température sans condensation d'eau est de 100 °C ou plus.

11. Procédé pour produire un oxyde de zinc particulaire ultrafin selon l'une quelconque des revendications 1 à 10, **caractérisé en ce que** la buse pour éjecter le gaz oxydant comprend une pluralité de buses.

12. Procédé pour produire un oxyde de zinc particulaire ultrafin selon l'une quelconque des revendications 1 à 11, comprenant l'étape supplémentaire consistant à revêtir de silice la surface de l'oxyde de zinc particulaire ultrafin.

13. Procédé pour produire un oxyde de zinc particulaire ultrafin selon l'une quelconque des revendications 1 à 11, comprenant l'étape supplémentaire consistant à formuler l'oxyde de zinc particulaire ultrafin sous la forme d'un matériau cosmétique, où le matériau cosmétique comprend de l'oxyde de zinc particulaire ultrafin en une quantité de 1 à 40 %.

# Fig. 1

Combustion supporting gas      Flammable gas      Carrier gas

Oxidizing gas containing
oxygen and water vapor

3

8

4

9

2

10

7

1

6

5

Product

EP 1 172 334 B1

**Fig. 2(a)**

First nozzle
Zinc vapor + carrier gas

Second nozzle
Oxidizing gas
($O_2$, $H_2O$, air)

1

Collection of powder

**Fig. 2(b)**

First nozzle
Zinc vapor + carrier gas

Second nozzle
Oxidizing gas
($O_2$, $H_2O$, air)

1

Third nozzle
Combustion exhaust gas
of flammable gas

Collection of powder

**Fig. 2(c)**

First nozzle
Zinc vapor + carrier gas

1

Third nozzle
Combustion exhaust gas
of flammable gas

Collection of powder

# Fig. 3

EP 1 172 334 B1

■ Example material (Invention)

▲ Comparative material

y+200%

y=-0.152Ln(A)+0.745

y-30%

Specific volume/Specific surface area (ml/m²)

Specific surface area (m²/g)

## Fig. 4

**Legend:**
- ▲ Example material 1
- ◇ Example material 2
- ◆ Example material 3
- △ Example material 4
- ● Comparative material 1
- ■ Comparative material 2
- ✕ Comparative material 3
- ☐ Comparative material 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• WO 9847476 A